# EUROPÄISCHE PATENTSCHRIFT

(11) **EP 1 892 230 B1**
(45) Veröffentlichungstag und Bekanntmachung des Hinweises auf die Patenterteilung: **13.04.2011**
(21) Anmeldenummer: 07015929.8
(22) Anmeldetag: 14.08.2007
(51) Int. Cl.: C07C 5/03, C07C 5/10

(54) **Verfahren und Vorrichtung zur Herstellung von Ethylcyclohexan durch katalytische Hydrierung in der Flüssigphase**
Method and device for manufacturing ethyl cyclohexane by catalytic hydrogenation in the liquid phase
Procédé et dispositif destinés à la fabrication d'éthylcyclohexane par hydrogénation catalytique en phase liquide

(30) Priorität: 17.08.2006 DE 102006038631
(43) Veröffentlichungstag der Anmeldung: 27.02.2008
(73) Patentinhaber: Miltitz Aromatics GmbH, 06766 Wolfen (DE)
(72) Erfinder: Müller, Peter, 04229 Leipzig (DE); Otto, Andreas, 06800 Jessnitz (DE); Doerfelt, Stephan, 08371 Glauchau (DE)
(74) Vertreter: Tragsdorf, Bodo

(56) Entgegenhaltungen:
- US-A- 5 811 595
- C.J.BOXWELL ET AL: "A Highly Selective Arene Hydrogenation Catalyst That Operates In Ionic Liquid" JACS, Bd. 124, 2002, Seiten 9334-9335, XP009093886
- M.FAURE ET AL.: "Catalytic Hydrogenation of Aromatics under Biphasic Conditions" JOURNAL OF ORGANOMETALLIC CHEMISTRY, Bd. 621, 2001, Seiten 103-108, XP004231242
- T.MIYAZAKI ET AL.: "Hydrogenation of Olefins with Cationic Rhodium Complex Intercalated in Fluoro Tetrasilicic Mica" CHEMISTRY LETTERS, 1985, Seiten 793-796, XP009093951

## Beschreibung

Die Erfindung betrifft ein Verfahren zur Herstellung von Ethylcyclohexan durch Hydrierung von mit Ethylbenzen verunreinigtem Ethylcyclohexan oder 4-Vinylcyclohexen an einem in der Flüssigphase suspendierten Edelmetallkatalysator und eine zur Durchführung des Verfahrens geeignete Vorrichtung.

Der vollständig gesättigte Kohlenwasserstoff Ethylcyclohexan (ECH) wird aufgrund seiner speziellen Eigenschaften als Lösungsmittel, insbesondere für apolare Stoffe, eingesetzt. ECH liegt in einem weiten Temperaturbereich von - -111°C (Festpunkt) bis 131 °C (Siedepunkt) in flüssiger Phase vor.

ECH wird technisch durch Hydrierung von 4-Vinylcyclohexen (VCH) gewonnen. Das Ausgangsprodukt VCH erhält man aus der Dimerisierung von Butadien.

Bei der technischen Hydrierung von VCH tritt als Konkurrenzreaktion zur Hydrierung auch die Freisetzung von Wasserstoff auf und es kommt zur Aromatisierung des Sechsrings unter Bildung von Ethylbenzen. Dies ist auch der Grund für einen relativ hohen Aromatengehalt von bis zu 25% im technischen ECH.

Bei der großtechnischen Herstellung ist es aus ökonomischer Sicht bisher nur möglich, ECH mit einer Reinheit von durchschnittlich 85% zu gewinnen. Der aus Ethylbenzen bestehende Rest, als Verunreinigung, ist für viele Anwendungen des ECH von Nachteil, sodass das Ethylbenzen entfernt werden muss.

Aus der DE-PS 1 184 756 ist ein Verfahren zur Hydrierung von aromatischen Kohlenwasserstoffen bekannt, bei dem das Zielprodukt Cyclohexan in hoher Reinheit erhalten werden soll. Die Hydrierung in erfolgt in zwei Stufen, wobei in einem ersten Reaktor die Hydrierung in flüssiger Phase an einem suspendierten, festen Katalysator erfolgt und in einer zweiten Stufe der aus dem ersten Reaktor austretende Gasstrom in einem zweiten Reaktor an einem festen Katalysatorbett in der Gasphase weiter bis zum Zielprodukt hydriert wird.

Diese Verfahrensweise einer Kombination von Flüssig- und Gasphasenhydrierung ist für eine großtechnische und wirtschaftliche Herstellung von ECH ungeeignet.

Aus DE 2 364 891 A ist es bekannt, ECH aus einem Gemisch von Ethylbenzol, m-Xylol sowie gegebenenfalls p-Xylol und/oder o-Xylol herzustellen, wobei eine im wesentlichen o-Xylol enthaltende Fraktion abgetrennt und der Rest des Gemisches partiell katalytisch hydriert wird, begrenzt auf 50 bis 99 % ECH und 10 bis 50 % Xylol. Aus dem in der ersten Stufe hydrierten Gemisch werden die nicht hydrierten Xylole destillativ abgetrennt und in einer zweiten Stufe wird der Rest des hydrierten Gemisches einer weiteren katalytischen Hydrierung unterworfen, um das restliche Ethylbenzol in ECH umzuwandeln.

Diese Verfahrensweise erfordert einen großen apparatechnischen Aufwand. Die Isolierung des ECH ist zeitaufwendig und kostenintensiv. Das erzielbare Durchsatzvolumen ist relativ gering.

Bekannt ist auch, dass die Hydrierverfahren zur Herstellung von Ethylcyclohexan Gleichgewichtsreaktionen sind, wobei je nach Reaktionsbedingungen unterschiedliche Mengen an Ethylcyclohexan entstehen können. Hohe Temperaturen (> 325 °C) sowie niedrige Wasserstoff-Partialdrücke begünstigen die Bildung von Ethylbenzen, niedrige Temperaturen (< 250 °C) und hohe Wasserstoff-Partialdrücke die von Ethylcyclohexan.

Der Erfindung liegt die Aufgabe zugrunde, ein Verfahren zur Herstellung von Ethylcyclohexan durch katalytische Hydrierung in der Flüssigphase zu schaffen, dass für eine großtechnische Umsetzung geeignet ist und mit dem ECH in hoher Reinheit kostengünstig hergestellt werden kann. Ferner soll eine zur Durchführung des Verfahrens geeignete Vorrichtung geschaffen werden.

Erfindungsgemäß wird die Aufgabe durch die im Anspruch 1 angegebenen Merkmale gelöst. Vorteilhafte Ausgestaltungen und Weiterbildungen der Verfahrensweise sind Gegenstand der Ansprüche 2 bis 11. Eine geeignete Vorrichtung zur Durchführung des Verfahrens ist im Anspruch 12 angegeben. Vorteilhafte Ausgestaltungen dieser Vorrichtung sind Gegenstand der Ansprüche 13 bis 15.

Gemäß der vorgeschlagenen Verfahrensweise wird die Reaktion in mehreren Reaktionszyklen in einem Schleifenreaktor durchgeführt, bestehend aus einer Anfahrphase und mehreren Reaktionszyklen.

Mit Beginn der Anfahrphase wird als Ausgangsprodukt eine erste Teilmenge an ECH vorgelegt.

Dabei kann es sich um technisches ECH handeln, das mit Ethylbenzen verunreinigt ist, oder um reines ECH, z.B. mit einem Ethylbenzen-Gehalt von unter 0,01 %. Auch zur Herstellung von reinem ECH aus 4-Vinylcyclohexen ist es unbedingt erforderlich, dass in der Anfahrphase ECH vorgelegt wird. Die eingesetzte Teilmenge muss mindestens so groß sein, dass diese innerhalb des Schleifenreaktors umgepumpt werden kann. Nachfolgend wird die erforderliche Menge an Katalysator zugegeben, der in einem Lösungsmittel, vorzugsweise Isopropanol, suspendiert ist. Der Zusatz an Katalysator erfolgt nur einmal, vor Beginn der Anfahrphase.

Danach wird inertisiert, vorzugsweise mit Stickstoff, und das Inertisierungsmittel vollständig durch Wasserstoff ausgetauscht. Wasserstoff wird ausschließlich über eine in den Schleifenreaktor eingebundene Dispergiervorrichtung, z.B. eine Strahlpumpe, eingetragen. Nach dem erstmaligen Eintrag von Wasserstoff wird das Reaktionsgemisch im Kreislauf umgepumpt. Gleichzeitig wird der Reaktor bis auf die erforderliche Reaktionstemperatur erwärmt. Wenn die Wasserstoffaufnahme, in der Regel nach mehreren Stunden, auf einen vorgegebenen Grenzwert abgesunken ist, wird die Anfahrphase beendet. Das Abbruchkriterium, die Abnahme des Wasserstoff-Volumenstromes, ist abhängig vom Nennvolumen des Schleifenreaktors. Der Grenzwert ist erreicht, wenn der Wasserstoff-Volumenstrom auf < 0,01 % seines anfänglichen Höchstwertes gefallen ist.

Mit Beendigung der Anfahrphase ist das Reaktionsgemisch auf Reaktionstemperatur erwärmt und der erforderliche Betriebsdruck, der Wasserstoff-Partialdruck, liegt an. Dieser wird über das Wasserstoffregelventil des Schleifenreaktors eingestellt.

Der nachfolgende erste Reaktionszyklus wird mit der Zudosierung der zweiten Teilmenge an Ausgangsprodukt begonnen. Als Ausgangsprodukt kann entweder technisches Ethylcyclohexan oder 4-Vinylcyclohexen zudosiert werden. Der Reaktorinhalt wird umgepumpt und nach einer Reaktionsdauer von mehreren Stunden wird der Reaktionszyklus abgebrochen, und zwar dann, wenn die Wasserstoffaufnahme auf den vorgegebenen Grenzwert abgesunken ist. Der Grenzwert entspricht dem der Anfahrphase. Unmittelbar nach Beendigung dieses Reaktionszyklus wird eine vorbestimmte Teilmenge an Hydrierprodukt über eine Metallfilterkerze ausgekreist. Der suspendierte Katalysator verbleibt im Kreislauf des Schleifenreaktors. Die nachfolgenden Reaktionszyklen verlaufen in analoger Weise. Die nach Beendigung eines Reaktionszyklus ausgekreiste Teilmenge an Hydrierprodukt wird im nachfolgenden Reaktionszyklus entweder durch Dosierung von frischem, technischen Ethylcyclohexan oder 4-Vinylcyclohexen ersetzt und der Reaktorinhalt weiter umgepumpt. Nach Erreichen des Grenzwertes für die Wasserstoffaufnahme wird der jeweilige Reaktionszyklus beendet.

Während der einzelnen Reaktionszyklen werden eine Reaktionstemperatur von 70 bis 130 °C und ein Betriebsdruck von 10 bis 20 bar eingehalten. Wasserstoff wird kontinuierlich über die in den Schleifenreaktor eingebundene Dispergiervorrichtung eingetragen, wodurch das Reaktionsgemisch intensiv verwirbelt wird. Die Reaktivität des Katalysators wird an Hand der gaschromatografischen Bestimmung der Komponenten der ausgekreisten Mengen an Hydrierprodukt überwacht. Bei stark nachlassender Reaktivität wird die Reaktion beendet. Das noch im Schleifenreaktor befindliche katalysatorhaltige Produkt wird abgelassen (Ablaufprodukt). Danach erfolgt dann ein neuer Ansatz mit Beginn der Anfahrphase.

Das nach den einzelnen Reaktionszyklen ausgekreiste Hydrierprodukt kann dann entweder als hochprozentiges ECH mit einem Gehalt an Ethylbenzen von < 0,01 % direkt seinem Verwendungszweck zugeführt werden oder nach Bedarf durch Destillation weiter gereinigt werden.

Mit dem vorgeschlagenen Verfahren kann hochprozentiges ECH in technischem Maßstab in besonders wirtschaftlicher Art und Weise hergestellt werden. Wahlweise kann innerhalb der einzelnen Reaktionszyklen entweder technisches ECH oder 4-Vinylcyclohexen zudosiert werden. Entgegen den Erwartungen war es möglich, die Gleichgewichtsreaktion bei geringen Temperaturen und geringem Betriebsdruck durchzuführen, wobei das Entstehen von Ethylbenzen wirksam unterdrückt werden konnte. Auch während der Auskreisung von Hydrierprodukt kam es zu keiner nachträglichen Dehydrierung.

Außerdem führte die Verfahrensweise zu einer Verlängerung der Katalysatorstandzeiten.

Die Hydrierung des Ausgangsproduktes erfolgte nahezu quantitativ im Kern und an den in mehreren Versuchreihen ausgekreisten Mengen an Hydrierprodukt wurde ein Gehalt an Ethylbenzen von unter 0,01 % ermittelt. Bezogen auf die eingesetzten Mengen an Katalysator (Trockensubstanz) konnten vergleichsweise hohe bis sehr hohe Durchsatzmengen erzielt werden, von beispielsweise 10 bis 15 kg Ethylbenzen/kg Katalysator und Stunde sowie durchschnittlich 40 kg 4-Vinylcyclohexen/kg Katalysator und Stunde.

In der Regel wird in der Anfahrphase technisches ECH vorgelegt. Möglich ist jedoch auch der Einsatz von ECH, das als Filtrat aus der vorhergehenden Hydrierung gewonnen wurde.

Falls 4-Vinylcyclohexen zu hochprozentigem ECH hydriert werden soll, so kann dieses entweder mit Beginn des ersten Reaktionszyklus oder während eines oder mehreren nachfolgender Reaktionzyklen zudosiert werden, jeweils als Austausch für die ausgekreiste Menge an Hydrierprodukt. Die ausgekreiste Menge an Hydrierprodukt kann vollständig oder auch nur teilweise oder im Überschuss ersetzt werden, wobei das vorhandene Füllvolumen des Schleifenreaktors zu berücksichtigen ist.

Bei Einsatz von 4-Vinylcyclohexen sollten vorzugsweise eine Reaktionstemperatur von 80 bis 100 °C und ein Druck von 8 bis 12 bar eingehalten werden. Da die Hydrierung von 4-Vinylcyclohexen stark exotherm verläuft, ist eine entsprechende Kühlung des Reaktionsgemisches erforderlich.

Die Hydrierung von technischem Ethylcyclohexan zu hochprozentigem Ethylcyclohexan wird vorzugsweise bei Temperaturen von 110 bis 120 °C und Drücken von 15 bis 20 bar durchgeführt.

Die nach den einzelnen Reaktionszyklen ausgekreiste Menge an Hydrierprodukt beträgt 40 bis 75% des Reaktorinhalts.

Als wirkungsvoll hat sich ein handelsüblicher Katalysator auf Ruthenium-Basis erwiesen, wobei das Verfahren auch mit anderen in der Flüssigphase suspendierbaren Katalysatoren durchgeführt werden kann.

Die Erfindung soll nachstehend an einigen Beispielen erläutert werden. In der zugehörigen Zeichnung ist eine Vorrichtung zu Durchführung des Verfahrens in vereinfachter schematischer Darstellung gezeigt.

Die Vorrichtung besteht aus einem Schleifenreaktor SR mit einer Kreislaufleitung 11, in die ein Reaktor 1, eine Umlaufpumpe 12, ein Wärmetauscher 13, eine Metallfilterkerze 14, eine Leitung 7 für den Eintrag von Ausgangsprodukt und eine Strahlpumpe 9 eingebunden sind.

Die Strahlpumpe 9 ist unmittelbar vor dem Kopf des Reaktors 1 angeordnet und ragt in diesen hinein.

Der Reaktor 1 ist zur Beheizung mittels Dampf mit einem Heizmantel 2 ausgerüstet. Am Kopf des Reaktors 1 ist in diesen eine über ein Ventil 4a absperrbare Leitung 4 mit einer Dosierschleuse zum Eintrag der Katalysatorsuspension eingebunden. Außerdem ist am Kopf des Reaktors 1 eine Leitung 5 zum Eintrag von Stickstoff und zur Entlüftung angeschlossen.

Über die Leitung 5a wird Stickstoff zugeführt und über die Leitung 5b entlüftet. In beide Leitungen 5a, 5b ist jeweils ein Ventil 6 eingebunden. Der Eintrag der im Kreislauf gefahrenen Reaktionsmischung in den Reaktor 1 erfolgt über die Strahlpumpe 9. Über diese wird auch der über die Leitung 8 zugeführte Wasserstoff eingetragen. Die Leitung 8 ist mittels eines Ventils 8a absperrbar. In diese ist eine mit dem Reaktor 1 verbundene Rücklaufleitung 10 für unverbrauchten Wasserstoff eingebunden. Der zugeführte Wasserstoff-Volumenstrom wird mittels einer Messeinrichtung FI gemessen. Zur Messung der Temperatur im Reaktor 1 ist eine Messeinrichtung TI vorgesehen.

Mit Beginn der Anfahrphase werden zuerst über die Leitung 7 und die Kreislaufleitung 11 eine erste Teilmenge technisches oder reines ECH und über die Leitung 4 die erforderliche Menge an suspendiertem Katalysator in den gereinigten Reaktor 1 eingetragen. Nach dem Verschließen der Ventile 7a und 4a wird inertisiert, wobei Stickstoff über die Leitungen 5a und 5 zugeführt und dieser anschließend gegen Wasserstoff ausgetauscht wird. Wasserstoff wird über die Leitung 8 und die Strahlpumpe 9 in den Reaktor 1 eingetragen. Anschließend wir die Umlaufpumpe 12 eingeschaltet, die Reaktionsmischung im Kreislauf gefahren und der Reaktor 1 langsam angeheizt. Über das Wasserstoffregelventil 8a wird der erforderliche Betriebsdruck eingestellt. Nach einer mehrstündigen Betriebsdauer, wenn die Wasserstoffaufnahme auf den vorgegebenen Abbruchwert abgesunken ist, wird die Anfahrphase beendet. Danach wird die zweite Teilmenge, entweder technisches ECH oder VCH, über die Leitung 7 zudosiert und der erste Reaktionszyklus begonnen. Dieser wird beendet, wenn die Wasserstoffaufnahme wieder auf den vorgegebenen Abbruchwert abgesunken ist. Anschließend wird eine Teilmenge an Hydrierprodukt 15 über die Filterkerze 14 ausgekreist und ein neuer Reaktionszyklus begonnen, durch Zudosieren einer entsprechenden Menge an technischem ECH oder VCH über die Leitung 7. In analoger Weise werden weitere Reaktionszyklen durchgeführt, wobei die jeweils ausgekreiste Menge an Hydrierprodukt durch technisches ECH oder VCH ersetzt wird. Die Reaktion wird nach einer bestimmten Anzahl an Reaktionszyklen beendet, und zwar dann, wenn anhand einer ausgekreisten Teilmenge der gaschromatografisch bestimmte Gehalt an Ethylbenzen einen vorgegebenen Wert, z. B. 0,01 Fläche-%, überschreitet. Mit Abschluss der Reaktion wird nach der letzten Auskreisung das noch im Reaktor 1 befindliche Ablaufprodukt über den Ablauf 3 abgelassen. Anschließend kann mit einer neuen Reaktion begonnen werden, die wie vorstehend erläutert mit der Anfahrphase eingeleitet wird.

Während der Zudosierung von VCH ist es erforderlich, die Kühleinrichtung 13 in Betrieb zu nehmen, da der Reaktionsverlauf exotherm ist.

Hinsichtlich weiterer Einzelheiten zur Verfahrensweise in wird auf die nachfolgenden Beispiele verwiesen.

### Beispiel 1 : Hydrierung von technischem Ethylcyclohexan (ECH)

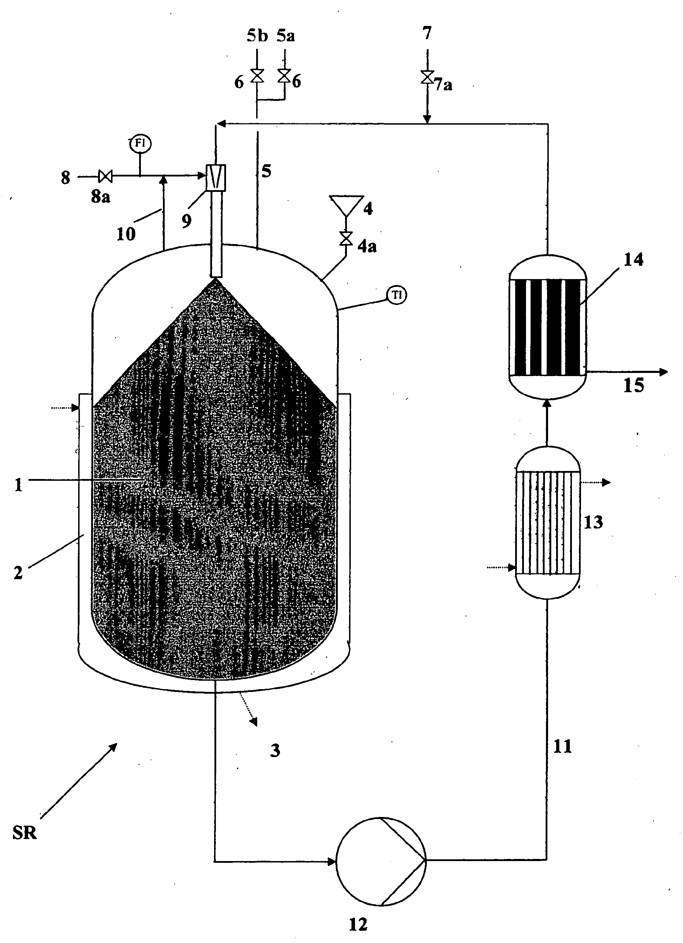

Die Hydrierung erfolgte in einem Schleifenreaktor SR, wie in der Zeichnung gezeigt, mit einem Nennvolumen von 800 Liter. Zunächst wurden während der Anfahrphase SB0 251 kg technisches Ethylcyclohexan mit ca. 20 % Restanteil von Ethylbenzen und 1,7 kg des in 15 Liter Isopropanol suspendierten, wasserfeuchten Ruthenium-Katalysators über eine in die Leitung 7 eingebundene Dosierschleuse in den gereinigten Reaktor 1 vorgelegt. Nach Verschließen des Ventile 4a und 7a, Inertisieren mit Stickstoff und Austausch des Stickstoffs gegen Wasserstoff wurde die Umlaufpumpe 12 eingeschaltet und das im Reaktor 1 befindliche Reaktionsgemisch umgepumpt. Nach Stabilisierung des Differenzdruckes vor und nach der Umlaufpumpe 12 wurde langsam angeheizt, wobei eine messbare Wasserstoffaufnahme ab einer Temperatur von ca. 80 °C einsetzte. Die Reaktionstemperatur wurde auf 120 °C und ein Arbeitsdruck von 11 bis 14 bar über das Wasserstoffregelventil 8a eingestellt. Der anfängliche Wasserstoff-Volumenstrom lag zwischen 20 und 25 Nm³/h. Wasserstoff wurde über die Strahlpumpe 9 in den Reaktor 1 eingetragen, wobei eine intensive Verwirbelung des Reaktionsgemisches erfolgte.

Nach etwa 6 Stunden war die Wasserstoffaufnahme auf den Grenzwert < 0,2 Nm³/h (Abbruchkriterium) abgefallen und damit die Anfahrphase SBO beendet.

An einer aus dem Probestutzen entnommenen Probe wurde gaschromatografisch der Restgehalt an Ethylbenzen bestimmt, der 0,009 Flächen-% betrug.

Mit Beginn des ersten Reaktionszyklus SB1 wurden weitere 272 kg technisches Ethylcyclohexan über die Leitung 7 in den Reaktor 1 gepumpt und kontinuierlich Wasserstoff über die Strahlpumpe 9 zugeführt und das Reaktionsgemisch im Kreislauf umgepumpt, unter Einhaltung der erforderliche Reaktionstemperatur von ca. 120 °C.

Der Hydriervorgang wurde bis zum Erreichen des Grenzwertes für die Wasserstoffaufnahme (< 0,2 Nm³/h) durchgeführt und der erste Reaktionszyklus SB1 beendet.

Anschließend wurde über Metallfilterkerze ca. 50% an Hydrierprodukt ausgekreist. Die restliche Menge an Reaktionsprodukt verblieb zusammen mit dem Katalysator im Reaktor.

An einer Probe des ausgekreisten Hydrierproduktes des ersten Reaktionszyklus (SB1) wurde gaschromatografisch der Restgehalt an Ethylbenzen ermittelt, der 0,009 Flächen-% betrug.

Nachfolgend wurden ohne Veränderung der Katalysatormenge weitere 22 Reaktionszyklen in analoger Weise durchgeführt.

In der Tabelle 1 sind zur Anfahrphase SB0 und den nachfolgenden Reaktionszyklen (SB1 bis SB 23) die Einsatzmengen an Ausgangsprodukt, die Verfahrensparameter (Temperatur, Druck, Reaktionszeit, Wasserstoffverbrauch) sowie die jeweils ausgekreisten Mengen an Hydrierprodukt und die ermittelten Werte für den Gehalt an Ethylbenzen angegeben.

Da der Reaktor während der Dosierung des technischen ECH ständig beheizt werden musste, war die Dosiergeschwindigkeit für ein kontrolliertes Abreagieren des enthaltenen Ethylbenzens unkritisch. Mit fortschreitender Zahl der während der einzelnen Reaktionszyklen durchgeführten Teilhydrierungen stieg allmählich die erforderliche Nachreaktionszeit bis zum Erreichen des vorgegebenen Grenzwertes für die Wasserstoffaufnahme als Abbruchkriterium.

Insgesamt wurden während einer Gesamtreaktionsdauer von 196,84 h 9750 kg technisches ECH mit einem Restgehalt von ca. 20% Ethylbenzen zu hochprozentigem Ethylcyclohexan (Gehalt an Ethylbenzen < 0,01 Flächen-%) hydriert. Die eingesetzte Katalysatormenge betrug 1,7 kg (wasserfeucht, Wassergehalt 55,9 %, d. h. ca. 0,93 kg Katalysator Trockensubstanz), suspendiert in 15 Liter Isopropanol. Der Verbrauch an Wasserstoff lag bei 1.375,47 Nm³. Die durchschnittliche Durchsatzmenge an Ethylbenzen (bezogen auf Trockensubstanz des eingesetzten Katalysators) lag bei 13,35 kg / kg * h.

Ausgehend von dem vorgegebenen Abbruchkriterium (Wasserstoff-Volumenstrom < 0,2 Nm³/h) für die einzelnen Reaktionszyklen zeigen die Werte in Tabelle 1, dass nach einer bestimmten Anzahl an Reaktionszyklen (ab SB9) die Reaktionszeiten langsam ansteigen.

Nach den 23 Reaktionszyklen wurde der Hydrierprozess abgebrochen, obwohl der Katatalysator noch eine ausreichende Aktivität für weitere Reaktionszyklen besaß.

Ab dem 5. Reaktionszyklus (SB5) wurden die Restgehalte an Ethylbenzen (EB) nur noch an Mischproben aus mehreren Reaktionszyklen bestimmt.

Nach Auskreisung der letzten Teilmenge an Hydrierprodukt, nach dem 23. Reaktionszyklus (SB 23), wurde eine Restmenge von 269 kg katalysatorhaltigem Ablaufprodukt über ein Bodenventil aus dem Schleifenreaktor abgelassen.

Auf der Basis dieses Versuches wurden aus der erhaltenen Menge Hydrierprodukt durch Filtration (ohne Destillation) zwei repräsentative Mischungen zusammengestellt, die folgenden Eigenschaftswerte aufweisen:

| Parameter | Mischung 1 bestehend aus SB1 bis SB 11 | Mischung 2 bestehend aus SB 12 bis SB 23 |
|---|---|---|
| GC: | Gehalt ECH: 98,24 Flächen-% | Gehalt ECH: 98,36 Flächen-% |
| | Gehalt EB: 0,009 Flächen-% | Gehalt EB: 0,008 Flächen-% |
| n_{D}²⁰:¹⁾ | 1,43276 | 1,43276 |
| d₄²⁰:²⁾ | 0,78743 | 0,78742 |

| | | |
|---|---|---|
| ¹⁾ Brechungsindex bei 20 °C bei 589 nm ²⁾ Dichte bei 20 °C [g / cm³] | | |

**Tabelle 1:**

| Reaktionszyklus | Einsatz ECH technisch [kg] | Reaktionstemperatur [°C] | Arbeitsdruck [bar] | Reaktionszeit [h] | Wasserstoff-Verbrauch [Nm³] | ECH hydriert ausgekreist [kg] | Gehalt Ethylbenzen [Fl.-%] |
|---|---|---|---|---|---|---|---|
| SB 0 | 251 | 124,0 | 13,5 | 5,83 | 43,02 | 0 | 0,009 |
| SB1 | 272 | 124,0 | 13,5 | 6,33 | 46,71 | 270 | 0,009 |
| SB2 | 400 | 122,0 | 13,1 | 7,50 | 59,94 | 405 | 0,009 |
| SB3 | 496 | 122,0 | 13,2 | 6,67 | 65,54 | 502 | 0,008 |
| SB4 | 471 | 121,0 | 13,2 | 6,42 | 63,24 | 476 | 0,008 |
| SB5 | 438 | 122,6 | 13,2 | 6,33 | 61,43 | 443 | 0,009 |
| SB6 | 475 | 122,4 | 11,8 | 6,67 | 63,86 | 481 | |
| SB7 | 458 | 124,2 | 12,8 | 6,50 | 61,13 | 464 | |
| SB8 | 444 | 121,8 | 12,8 | 6,33 | 62,30 | 449 | 0,008 |
| SB9 | 423 | 120,8 | 10,8 | 7,75 | 59,50 | 428 | |
| SB10 | 448 | 119,8 | 13,0 | 8,25 | 62,90 | 453 | |
| SB11 | 409 | 121,8 | 12,7 | 7,42 | 58,96 | 413 | |
| SB12 | 467 | 122,0 | 12,1 | 7,83 | 63,32 | 472 | |
| SB13 | 465 | 122,5 | 12,8 | 8,58 | 62,80 | 470 | 0,008 |
| SB14 | 379 | 120,7 | 13,4 | 8,33 | 53,16 | 384 | |
| SB15 | 370 | 120,7 | 13,1 | 10,17 | 57,11 | 374 | |
| SB16 | 367 | 123,4 | 12,9 | 9,25 | 54,48 | 371 | 0,008 |
| SB17 | 417 | 122,1 | 13,0 | 11,42 | 60,75 | 422 | |
| SB18 | 425 | 122,8 | 14,4 | 10,08 | 59,56 | 430 | |
| SB19 | 404 | 122,7 | 14,4 | 9,67 | 56,77 | 409 | |
| SB20 | 425 | 120,4 | 14,1 | 11,67 | 57,65 | 430 | 0,008 |
| SB21 | 379 | 118,9 | 14,1 | 11,42 | 53,46 | 384 | |
| SB22 | 484 | 120,9 | 14,2 | 12,67 | 63,05 | 489 | |
| SB23 | 183 | 123,2 | 14,0 | 10,08 | 24,83 | 185 | |
| Gesamt | 9 750 | | | 196,84 | 1 375,47 | 9 604 | |
| Ablaufprodukt | | | | | | 269 | |

### Beispiel 2: Hydrierung von 4-Vinylcyclohexen zu Ethylcyclohexan (ECH)

Die Hydrierung erfolgte in einem Schleifenreaktor SR, wie in der Zeichnung gezeigt, mit einem Nennvolumen von 20 Liter.

Zunächst wurden mit Beginn der Anfahrphase SB0 11,35 kg technisches Ethylcyclohexan mit ca. 20 % Ethylbenzen sowie 69 g des in 500 ml Isopropanol suspendierten, wasserfeuchten Ruthenium-Katalysators in den gereinigten Reaktor 1 vorgelegt. Nach Verschließen des Reaktors 1, Inertisieren mit Stickstoff und Austausch gegen Wasserstoff wurde die Umlaufpumpe 12 eingeschaltet und nach Stabilisierung des Differenzdruckes vor und nach der Umlaufpumpe 12 stufenweise angeheizt, wobei eine messbare Wasserstoffaufnahme ab ca. 80 °C zu verzeichnen war. Die Reaktionstemperatur wurde auf 115 °C erhöht und ein Arbeitsdruck von 15 bar über das Wasserstoffregelventil 8a eingestellt. Der anfängliche Wasserstoff-Volumenstrom lag dann zwischen 11 und 13 Normliter/min. Nach 7,5 Stunden war der Wasserstoff-Volumenstrom auf 0,06 bis 0,10 Normliter/min gesunken (Abbruchkriterium). Aufgrund des geringen Nennvolumens des Schleifenreaktors war es erforderlich, bereits nach Beendigung der Anfahrphase eine Teilmenge an Hydrierprodukt auszukreisen. In diesem Fall wurden ca. 60% an im Reaktor befindlichem Hydrierprodukt über die Metallfilterkerze 14 ausgekreist. Der gaschromatografisch bestimmte Restgehalt an Ethylbenzen war 0,009 Flächen-%.

Nach Beendigung der Anfahrphase SB0 wurde mit dem ersten Reaktionszyklus SB1 begonnen. Zunächst wurden die Reaktortemperatur auf 90 °C und der Arbeitsdruck auf 12 bar abgesenkt. Anschließend wurden innerhalb von 5,5 Stunden 6,2 kg 4-Vinylcyclohexen gleichmäßig über die Leitung 7 zudosiert, wobei sofort eine kräftige Wasserstoffaufnahme (anfänglicher Wasserstoff-Volumenstrom von 12 bis 15 Normliter/min) zu verzeichnen war.

Der über die Leitung 8 zugeführte Wasserstoff-Volumenstrom wurde auf einen Wert von ca. 12 Normliter/min eingestellt. Aufgrund der stark exotherm einsetzenden Reaktion wurde die freiwerdende Reaktionswärme kontinuierlich über den Wärmetauscher 13 abgeführt, um zu verhindern, dass die Reaktionstemperatur 95 °C übersteigt. Etwa 90 Minuten nach Dosierende war der Wasserstoff-Volumenstrom auf den Grenzwert von < 0,1 Normliter/min gesunken und damit der erste Reaktionszyklus SB1 beendet. Es wurde mit der Auskreisung einer Teilmenge (6,55 kg) an Hydrierprodukt über die Metallfilterkerze14 begonnen. An einer Probe des ausgekreisten Hydrierproduktes wurden gaschromatografisch die Gehalte an Ethylcyclohexan (über 97 Flächen-%) und Ethylbenzen (0,001 Flächen-%) ermittelt. Anschließend wurde im nachfolgenden Reaktionszyklus (SB2) der Arbeitsdruck weiter auf 10 bar abgesenkt, mit der Dosierung der nächsten Teilmenge 4-Vinylcyclohexen (9,70 kg), als Austausch für das zuvor ausgekreiste Hydrierprodukt, begonnen und der Reaktionszyklus analog wie im ersten Reaktionszyklus SB1 durchgeführt. Nach erfolgter Auskreisung einer weiteren Teilmenge an Hydrierprodukt wurden noch 5 weitere Reaktionszyklen (SB3 bis SB7) durchgeführt.

Die gaschromatografisch ermittelten Werte für Ethylbenzen lagen alle unter 0,01 Flächen-%. Nach Auskreisung der letzten Teilmenge an Hydrierprodukt nach Beendigung des 7. Reaktionszyklus SB 7 über die Metallfilterkerze wurde eine Restmenge von 3,5 kg katalysatorhaltigem Ablaufprodukt über das Bodenventil des Reaktors abgelassen.

In der nachfolgenden Tabelle 2 sind zur Anfahrphase SB0 und den nachfolgenden Reaktionszyklen (SB1 bis SB 7) die Einsatzmengen an Ausgangsprodukt, die Verfahrensparameter (Temperatur, Druck, Reaktionszeit, Wasserstoffverbrauch) sowie die jeweils ausgekreisten Mengen an Hydrierprodukt und die ermittelten Werte für den Gehalt an Ethylbenzen angegeben.

Insgesamt wurden wurden als Ausgangsprodukt 11,35 kg Ethylcyclohexan technisch mit einem Ethylbenzen-Gehalt von > 20 % (in der Anfahrphase vorgelegt) und 60,0 kg 4-Vinylcyclohexen eingesetzt. Mit 69 g wasserfeuchtem Katalysator (Wassergehalt 55,9 %, d.h. 34,3 g Katalysator Trockensubstanz) konnte das Ausgangsprodukt nahezu quantitativ zu Ethylcyclohexan (70,25 kg) mit einem Gehalt an Ethylbenzen von < 0,01 Flächen-% hydriert werden. Die Gesamtreaktionszeit wurde hauptsächlich durch die Dosierzeit bestimmt, die erforderlich war, um die freiwerdende Reaktionswärme adäquat abzuführen.

Während die Durchsatzmenge an Ethylbenzen (bezogen auf Trockensubstanz des eingesetzten Katalysators) bei 10 kg / kg * h lag, betrug dieser bei der Verwendung von 4-Vinylcyclohexen durchschnittlich 39,9 kg / kg * h.

Auf der Basis dieses Versuches wurden aus den ausgekreisten Teilmengen an hydriertem ECH (SB0 bis SB7 gemäß Tabelle 2) durch Filtration (ohne Destillation) eine Mischung zusammengestellt, die folgende Eigenschaftswerte aufweist:

| Parameter | Mischung bestehend aus SB0 bis SB7 |
|---|---|
| GC: | Gehalt ECH: 97,4 Flächen-% |
| | Gehalt EB: 0,002 Flächen-% |
| n_{D}²⁰: | 1,43276 |
| d₄ ²⁰: | 0,7876 |

**Tabelle 2:**

| Semibatch-Nr. | Einsatz VCH [kg] | Reaktionstemperatur [°C] | Arbeitsdruck [bar] | Reaktions-Zeit [h] | Wasserstoff-Verbrauch [Normliter] | ECH hydriert ausgekreist [kg] | Gehalt Ethylbenzen [Fl.-%] |
|---|---|---|---|---|---|---|---|
| SB0 | 11,35¹⁾ | 115 | 15 | 7,5 | 1 596 | 7,00 | 0,009 |
| SB1 | 6,20 | 90 | 12 | 7,0 | 2722 | 6,55 | 0,001 |
| SB2 | 9,70 | 90 | 10 | 7,5 | 4089 | 10,20 | < 0,001 |
| SB3 | 8,35 | 90 | 10 | 6,5 | 3 501 | 8,80 | < 0,001 |
| SB4 | 8,40 | 90 | 10 | 6,5 | 3 549 | 8,85 | 0,001 |
| SB5 | 9,10 | 90 | 10 | 7,2 | 3 826 | 9,60 | 0,002 |
| SB6 | 9,05 | 90 | 10 | 7,2 | 3816 | 9,55 | 0,002 |
| SB7 | 9,20 | 90 | 10 | 7,5 | 3843 | 9,70 | 0,003 |
| Gesamt | 71,35 | | | 56,9 | 26 942 | 70,25 | |
| Ablaufprodukt | | | | | | 3,50 | |

| | | | | | | | |
|---|---|---|---|---|---|---|---|
| ¹⁾ technisches ECH | | | | | | | |

## Patentansprüche

1. Verfahren zur Herstellung von Ethylcyclohexan durch Hydrierung von mit Ethylbenzen verunreinigtem Ethylcyclohexan (technisches Ethylcyclohexan) oder 4-Vinylcyclohexen an einem in der Flüssigphase suspendierten Edelmetallkatalysator, **dadurch gekennzeichnet, dass** die Reaktion in mehreren Reaktionszyklen in einem Schleifenreaktor durchgeführt wird, wobei
a) in einer Anfahrphase als Ausgangsprodukt eine erste Teilmenge an ECH vorgelegt, Katalysator zugegeben, inertisiert und das Inertisierungsmittel durch Wasserstoff ausgetauscht wird, das Reaktionsgemisch im Kreislauf umgepumpt, der Reaktor auf Reaktionstemperatur erhitzt und die Anfahrphase beendet wird, wenn die Wasserstoffaufnahme auf einen vorgegebenen Grenzwert abgesunken ist;
b) mit Beginn des nachfolgenden ersten Reaktionszyklus der Reaktor mit der zweiten Teilmenge an Ausgangsprodukt, entweder technisches Ethylcyclohexan oder 4-Vinyl-cyclohexen, aufgefüllt wird, der Reaktorinhalt umgepumpt und der Reaktionszyklus beendet wird, wenn die Wasserstoffaufnahme auf den vorgegebenen Grenzwert abgesunken ist, wobei unmittelbar nach Beendigung dieses Reaktionszyklus eine vorbestimmte Teilmenge an Hydrierprodukt ausgekreist wird;
c) während aller weiteren Reaktionszyklen die nach dem vorangegangenen Reaktionszyklus ausgekreiste Teilmenge an Hydrierprodukt entweder durch technisches Ethylcyclohexan oder 4-Vinylcyclohexen ersetzt wird, der Reaktorinhalt umgepumpt und nach Erreichen des Grenzwertes für die Wasserstoffaufnahme der jeweilige Reaktionszyklus beendet wird;
und während der einzelnen Reaktionszyklen eine Reaktionstemperatur von 60 bis 130 °C und ein Druck von 5 bis 20 bar eingehalten werden, Wasserstoff über eine in den Schleifenreaktor eingebundene Dispergiervorrichtung eingetragen, das Reaktionsgemisch verwirbelt wird und die Reaktivität des Katalysators überwacht und bei stark nachlassender Reaktivität des Katalysators die Reaktion beendet wird.

2. Verfahren nach Anspruch 1, **dadurch gekennzeichnet, dass** in der Anfahrphase entweder technisches oder reines ECH vorgelegt wird.

3. Verfahren nach einem der Ansprüche 1 oder 2, **dadurch gekennzeichnet, dass** nach Beendigung der Anfahrphase eine Teilmenge an Hydrierprodukt ausgekreist wird.

4. Verfahren nach einem der Ansprüche 1 bis 3, **dadurch gekennzeichnet, dass** bei Einsatz von 4-Vinylcyclohexen dieses mindestens während eines Reaktionzyklus als Austausch für die ausgekreiste Menge an Hydrierprodukt zugesetzt wird

5. Verfahren nach einem der Ansprüche 1 bis 4, **dadurch gekennzeichnet, dass** bei Einsatz von 4-Vinylcyclohexen eine Reaktionstemperatur von 60 bis 110 °C und ein Druck von 5 bis 10 bar eingehalten werden, wobei das Reaktionsgemisch gekühlt wird.

6. Verfahren nach einem der Ansprüche 1 bis 4, **dadurch gekennzeichnet, dass** die Hydrierung von technischem Ethylcyclohexan zu hochprozentigem Ethylcyclohexan bei Temperaturen von 70 bis 130 °C und Drücken von 10 bis 20 bar durchgeführt wird.

7. Verfahren nach einem der Ansprüche 1 bis 6, **dadurch gekennzeichnet, dass** der Grenzwert für die Wasserstoffaufnahme, zur Beendigung der Anfahrphase oder der einzelnen Reaktionszyklen, weniger als 0,01 % des anfänglichen Wasserstoff-Volumenstroms beträgt.

8. Verfahren nach einem der Ansprüche 1 bis 7, **dadurch gekennzeichnet, dass** die mit Beginn der Anfahrphase zugeführte Teilmenge mindestens so groß ist, dass diese innerhalb des Schleifenreaktors eine Kreislauf-Fahrweise gewährleistet.

9. Verfahren nach einem der Ansprüche 1 bis 8, **dadurch gekennzeichnet, dass** die nach den Reaktionszyklen ausgekreiste Menge an Hydrierprodukt 40 bis 75% des Reaktorinhalts beträgt.

10. Verfahren nach einem der Ansprüche 1 bis 9, **dadurch gekennzeichnet, dass** als Katalysator ein Katalysator auf Ruthenium-Basis eingesetzt wird, der in einem alkoholischen Lösungsmittel suspendiert ist.

11. Verfahren nach einem der Ansprüche 1 bis 10, **dadurch gekennzeichnet, dass** Wasserstoff über eine in den Schleifenreaktor eingebundene Strahlpumpe eingetragen wird.

12. Vorrichtung zur Durchführung des Verfahrens nach mindestens einem der vorhergehenden Ansprüche, **dadurch gekennzeichnet, dass** diese aus einem Schleifenreaktor (SR) besteht, in dessen Kreislaufleitung (11) ein beheizbarer Reaktor (1), eine Umlaufpumpe (12), ein Wärmetauscher (13), eine Metallfilterkerze (14) zum Austragen von hydriertem Produkt, eine Zuführung (7) für Ausgangsprodukt und eine Strahlpumpe (9) eingebunden sind, wobei die Strahlpumpe (9) am Kopf des Reaktors (1) angeordnet ist und in diesen hineinragt und mit einer Leitung (8) zur Zuführung von Wasserstoff in Verbindung steht, wobei diese Leitung (8) mit einer Leitung (10) zur Rückführung von unverbrauchtem Wasserstoff aus dem Reaktor (1) verbunden ist, und in den Reaktor (1) eine Leitung (4) zur Zuführung eines in flüssiger Phase suspendierbaren Katalysators sowie eine Leitung (5) zur Zuführung eines Inertisierungsmittels eingebunden sind.

13. Vorrichtung nach Anspruch 12, **dadurch gekennzeichnet, dass** in die Leitung (5) zur Zuführung des Inertisierungsmittels zwei über Ventile (6) absperrbare Leitungen eingebunden sind, eine Entlüftungsleitung (5b) und eine Einspeiseleitung (5a) für das Inertisierungsmittel.

14. Vorrichtung nach einem der Ansprüche 12 oder 13, **dadurch gekennzeichnet, dass** in die Leitung (7) zur Zuführung des Ausgangsproduktes und in die Leitung (8) zur Einleitung des Wasserstoffs jeweils ein Ventil (7a, 8a) eingebunden ist.

15. Vorrichtung nach einem der Ansprüche 12 bis 13, **dadurch gekennzeichnet, dass** über das in die Leitung (8) eingebundene Ventil (8a) der Betriebsdruck im Schleifenreaktor (SR) einstellbar ist.

## Claims

1. Process for manufacturing ethylcyclohexane by hydrogenation of ethylcyclohexane contaminated with ethylbenzene (technical-grade ethylcyclohexane) or 4-vinylcyclohexene on a precious metal catalyst suspended in the liquid phase, **characterised in that** the reaction is carried out in several reaction cycles in a loop reactor, wherein
a) an initial partial quantity of ECH is added as starting product in a start-up phase, the catalyst is added, inerting is carried out with an inert gas and the inerting agent is replaced by hydrogen, the reaction mixture is circulated in the loop, the reactor is heated to reaction temperature and the start-up phase is terminated when the uptake of hydrogen has fallen to a preset threshold value;
b) the reactor is filled with the second partial quantity of starting product - either technical-grade ethylcyclohexane or 4-vinylcyclohexene - at the start of the subsequent initial reaction cycle, the contents of the reactor are circulated and the reaction cycle is terminated when the uptake of hydrogen has fallen to the preset threshold value, wherein a preset partial quantity of hydrogenation product is discharged immediately after the termination of this reaction cycle;
c) the partial quantity of hydrogenation product discharged after the previous reaction cycle is replaced either by technical-grade ethylcyclohexane or 4-vinylcyclohexene during all further reaction cycles, the contents of the reactor are circulated and the respective reaction cycle is terminated after the threshold value for the uptake of hydrogen has been reached;
and a reaction temperature of 60 to 130°C and a pressure of 5 to 20 bar are maintained during the individual reaction cycles, hydrogen is fed in by a dispersion device integrated into the loop reactor, the reaction mixture is swirled and the reactivity of the catalyst is monitored and the reaction is terminated in the case of strongly decreasing reactivity of the catalyst

2. Process according to claim 1, **characterised in that** either technical-grade or pure ECH is added in the start-up phase.

3. Process according to one of the claims 1 or 2, **characterised in that** a partial quantity of hydrogenation product is discharged after the termination of the start-up phase.

4. Process according to one of the claims 1 to 3, **characterised in that** when 4-vinylcyclohexene is used it is added during at least one reaction cycle as a replacement for the quantity of hydrogenation product discharged.

5. Process according to one of the claims 1 to 4, **characterised in that** when 4-vinylcyclohexene is used a reaction temperature of 60 to 110°C and a pressure of 5 to 10 bar are maintained, with the reaction mixture being cooled.

6. Process according to one of the claims 1 to 4, **characterised in that** technical-grade ethylcyclohexane is hydrogenated to high percentage ethylcyclohexane at temperatures of 70 to 130°C and pressures of 10 to 20 bar.

7. Process according to one of the claims 1 to 6, **characterised in that** the threshold value for the uptake of hydrogen for terminating the start-up phase or the individual reaction cycles amounts to less than 0.01% of the initial hydrogen flow rate.

8. Process according to one of the claims 1 to 7 **characterised in that** the partial quantity added at the beginning of the start-up phase is at least sufficiently large to ensure its circulation within the loop reactor.

9. Process according to one of the claims 1 to 8, **characterised in that** the quantity of hydrogenation product discharged after the reaction cycles amounts to at least 40 to 75% of the contents of the reactor.

10. Process according to one of the claims 1 to 9, **characterised in that** a ruthenium-based catalyst is used as a catalyst suspended in an alcoholic solvent.

11. Process according to one of the claims 1 to 10, **characterised in that** hydrogen is fed in by a jet pump integrated into the loop reactor.

12. Device for carrying out the process according to at least one of the previous claims, **characterised in that** said device comprises a loop reactor (SR) in whose circuit line (11), a heatable reactor (1), a circulating pump (12), a heat exchanger (13), a metal filter candle (14) for discharging the hydrogenation product, a feed (7) for the starting product and a jet pump (9) are integrated, with the jet pump (9) arranged at the head of the reactor (1) and protruding into said reactor and connected to a line (8) for feeding in hydrogen, wherein this line (8) is connected to a line (10) for the recirculation of non-consumed hydrogen from the reactor (1), and a line (4) for adding a catalyst suspendable in the liquid phase as well as a line (5) for feeding in an inerting agent are integrated into the reactor (1).

13. Device according to claim 12, **characterised in that** two lines, a vent line (5b) and a feeder line (5a) for the inerting agent, that can be shut off by valves (6), are integrated into the line (5) for feeding in the inerting agent.

14. Device according to one of the claims 12 or 13, **characterised in that** a valve (7a, 8a) is integrated respectively into the line (7) for adding the starting product and the line (8) for feeding in the hydrogen.

15. Device according to one of the previous claims 12 to 13, **characterised in that** the operating pressure in the loop reactor (SR) can be adjusted by the valve (8a) integrated in the line (8).

## Revendications

1. Procédé pour la fabrication d'éthylcyclohexane par hydrogénation d'éthylcyclohexane (éthylcyclohexane technique) pollué par de l'éthybenzène ou du 4-vinylcyclohexène sur un catalyseur de métaux nobles en suspension en phase liquide, **caractérisé en ce que** la réaction est réalisée dans une colonne à écoulement en boucle en plusieurs cycles réactionnels,
a) dans une phase de démarrage, une première quantité partielle de ECH est présentée sous forme de produit initial, ajoutée au catalyseur, inertisée, et l'agent d'inertisation est remplacé par de l'hydrogène, le mélange réactionnel est recyclé par pompage, la colonne est portée à la température réactionnelle et la phase de démarrage est terminée lorsque l'absorption d'hydrogène est descendue à une valeur limite prédéfinie;
b) une fois le premier cycle réactionnel suivant commencé, la colonne est remplie avec la seconde quantité partielle de produit initial, soit de l'éthylcyclohexane technique soit du 4-vinylcyclohexène, le contenu de la colonne est recyclé par pompage et le cycle réactionnel est terminé lorsque l'absorption d'hydrogène est descendue à la valeur limite prédéfinie, une quantité partielle prédéfinie de produit d'hydrogénation étant évacuée du circuit immédiatement à la fin de ce cycle réactionnel ;
c) au cours de tous les autres cycles réactionnels, la quantité partielle de produit d'hydrogénation, évacuée du circuit au terme du cycle réactionnel précédent, est remplacée soit par l'éthylcyclohexane technique soit par le 4-vinylcyclohexène, le contenu réactionnel est recyclé par pompage et une fois la valeur limite d'absorption d'hydrogène atteinte, le cycle réactionnel correspondant est terminé ;
et au cours de chacun des cycles réactionnels, une température réactionnelle comprise entre 60 et 130°C et une pression comprise entre 5 et 20 bar sont maintenues, de l'hydrogène est introduit par le biais d'un dispositif de dispersion intégré dans la colonne à écoulement en boucle, le mélange réactionnel est brassé, et la réactivité du catalyseur est surveillée et la réaction est terminée en cas de réactivité du catalyseur fortement décroissante.

2. Procédé selon la revendication 1, **caractérisé en ce que** soit du ECH technique soit du ECH pur est présenté en phase de démarrage.

3. Procédé selon l'une des revendications 1 ou 2, **caractérisé en ce qu'**au terme de la phase de démarrage une quantité partielle de produit d'hydrogénation est évacuée du circuit.

4. Procédé selon l'une des revendications 1 à 3, **caractérisé en ce qu'**en cas d'utilisation de 4-vinylcyclohexène, celui-ci est rajouté pendant au moins un cycle réactionnel sous forme d'échange destiné à la quantité de produit hydrogéné, évacuée du circuit.

5. Procédé selon l'une des revendications 1 à 4, **caractérisé en ce qu'**en cas d'utilisation de 4-vinylcyclohexène, une température réactionnelle comprise entre 60 et 110°C et une pression comprise entre 5 et 10 bar sont maintenues, le mélange réactionnel étant refroidi.

6. Procédé selon l'une des revendications 1 à 5, **caractérisé en ce que** l'hydrogénation d'éthylcyclohexane technique en éthylcyclohexane à teneur élevée est réalisée à des températures comprises entre 70 et 130°C et des pressions comprises entre 10 et 20 bar.

7. Procédé selon l'une des revendications 1 à 6, **caractérisé en ce que** la valeur limite d'absorption d'hydrogène pour terminer la phase de démarrage ou chacun des cycles réactionnels, est inférieure à 0,01 % du courant volumique d'hydrogène initial.

8. Procédé selon l'une des revendications 1 à 7, **caractérisé en ce que** la quantité partielle amenée au début de la phase de démarrage est au moins en quantité suffisante pour garantir un mode de procédé en boucle à l'intérieur de la colonne à écoulement en boucle.

9. Procédé selon l'une des revendications 1 à 8, **caractérisé en ce que** la quantité de produit d'hydrogénation évacuée après les cycles réactionnels correspond à 40 jusqu'à 75% du contenu de la colonne.

10. Procédé selon l'une des revendications 1 à 9, **caractérisé en ce qu'**on utilise comme catalyseur un catalyseur à base de ruthénium en suspension dans un solvant alcoolique.

11. Procédé selon l'une des revendications 1 à 10, **caractérisé en ce que** de l'hydrogène est introduit par le biais d'un éjecteur intégré dans la colonne d'écoulement en boucle.

12. Dispositif pour la mise en oeuvre du procédé selon au moins l'une des revendications précédentes, **caractérisé en ce que** celui-ci se compose d'une colonne à écoulement en boucle (SR) dans la conduite de circuit (11) duquel sont intégrés une colonne chauffante (1), une pompe à circulation (12), un échangeur de chaleur (13), une bougie filtrante en métal (14) pour l'évacuation du produit d'hydrogénation, une conduite d'arrivée (7) pour le produit initial et un éjecteur (9), l'éjecteur (9) étant disposé à la tête de la colonne (1), pénétrant à l'intérieur de celle-ci et étant relié à une conduite (8) d'arrivée de l'hydrogène, cette conduite (8) étant reliée à une conduite (10) pour le retour de l'hydrogène non utilisé venant de la colonne (1), et une conduite (4) d'amenée d'un catalyseur en suspension dans une phase liquide ainsi qu'une conduite (5) de retour d'un agent d'inertisation étant intégrées dans la colonne (1).

13. Dispositif selon la revendication 12, **caractérisé en ce que** deux conduites pouvant être fermées par des soupapes (6) sont intégrées dans la conduite (5) d'arrivée de l'agent d'inertisation, une conduite de purge (5b) et une conduite d'alimentation (5a) pour l'agent d'inertisation.

14. Dispositif selon l'une des revendications 12 ou 13, **caractérisé en ce qu'**une soupape (7a, 8a) est intégrée à la fois dans la conduite (7) d'arrivée du produit initial et dans la conduite (8) d'introduction de l'hydrogène.

15. Dispositif selon l'une des revendications 12 à 13, **caractérisé en ce que** la pression de service peut être réglée dans la colonne à écoulement en boucle (SR) par le biais de la soupape (8a) intégrée dans la conduite (8).
